# EUROPEAN PATENT SPECIFICATION

(11) **EP 3 160 360 B1**
(45) Date of publication and mention of the grant of the patent: **27.11.2019**
(21) Application number: 15729927.2
(22) Date of filing: 15.06.2015
(51) Int. Cl.: A61B 10/00

(54) **LIQUID SAMPLE COLLECTION DEVICE**
VORRICHTUNG ZUR ENTNAHME FLÜSSIGER PROBEN
DISPOSITIF DE COLLECTE D'UN ÉCHANTILLON LIQUIDE

(30) Priority: 27.06.2014 GB 201411491
(43) Date of publication of application: 03.05.2017
(73) Proprietor: Randox Laboratories Ltd., Crumlin, Co. Antrim Northern Ireland, BT29 4QY (GB)
(72) Inventor: JACKSON, Stuart, Co Antrim, Northern Ireland BT29 4QY (GB); MCCONNELL, Ivan, Co Antrim, Northern Ireland BT29 4QY (IE); LAMONT, John, Co Antrim, Northern Ireland BT29 4QY (GB); FITZGERALD, Stephen Peter, Co Antrim, Northern Ireland BT29 4QY (IE)
(74) Representative: Gill Jennings & Every LLP
(86) International application number: PCT/GB2015/051749
(87) International publication number: WO 2015/198019

(56) References cited:
- EP-A2- 0 289 761
- CH-A- 533 989
- JP-A- S63 302 321
- US-A- 999 053
- US-A- 4 495 951
- US-A1- 2013 302 791

## Description

### Field of the Invention

The invention relates to a liquid sample collection device for targeting, separating and isolating a specific portion of a continuous stream of liquid to be collected.

### Description of Background Art

Biomarkers, micro-organisms, proteins, DNA and chemicals are often suspended in a liquid or transported by a liquid flow. It is often desirable to collect such liquids for storage, processing or to detect the presence, concentration, state, or other property of the suspended matter. Collecting such a liquid often requires instigating a flow or stream of the liquid from its reservoir to a collection receptacle. An example of a liquid containing suspended matter which is collected from a liquid stream is urine.

Traditionally urine is collected by asking the patient to fill a cup to a level marked on the side. This is often imprecise, and it is common for patients to overfill or underfill the cup. Furthermore, in urine collection for STI (sexually transmitted infection) testing, the first part of the stream is regarded as that which provides the most useful sample, containing the highest concentration of suspended matter detected in STI tests. Collecting and isolating this first portion of the stream would therefore lead to improved sensitivity and specificity of testing. Conversely, it may be desirable to target a later portion of the liquid stream, in which there is a lower concentration of suspended matter detected in STI testing.

It is therefore desirable to allow for sample collection which targets a specific portion of a liquid stream and ensures that the targeted portion is isolated to prevent dilution by liquid from other portions of the stream.

A number of liquid sample collection devices which target specific portions of a liquid stream are known.

US 2006/0184064 A1 discloses a urine collection device which redirects a first portion of the urine flow and collects a "mid-stream" portion of the urine flow. The device comprises a support apparatus which, in use, sits on top of a conventional toilet seat. The apparatus features a flow divider which comprises a chamber with a hole, through which the first portion of the urine steam flows. The rate at which urine flows through the hole is much slower than the typical rate that urine enters the chamber, leading to an increasing liquid level. As the liquid level increases, it overflows the chamber and is directed to an attachment section where a sample cup collects the overflow.

US 2006/0184064 A1 comprises an overflow chamber, in which the urine to be collected mixes before overflowing into the sample cup thereby resulting in contamination of different portions of the stream.

WO 2004/010873 A1 discloses a liquid sampler for sampling a first portion of a liquid flow. The sample entering the device passes through a valve, which closes once a predetermined volume of liquid is collected. Excess liquid is then directed to waste.

WO 2010/058210 A1 discloses a device for collecting first pass urine. The device comprises an opening for collecting a urine sample and directing it downstream where a circular orifice opens into a collecting vessel. Urine entering the device flows into the collecting vessel and begins to fill the collection vessel with a first portion of the urine. As the collection vessel fills, a collection member, which floats on the urine, rises up inside the vessel until it blocks the circular orifice into the collection vessel, thereby isolating the first portion of the urine sample from any further urine entering the device.

Both of WO 2004/010873 A1 and WO 2010/058210 A1 use moving parts which are difficult to manufacture, may contaminate the sample and to which the sample may stick following collection.

An example of a liquid sample collection device according to the preamble of claim 1 may be found in JP S63302321 A.

It is therefore desirable to create a liquid sample collection device which does not suffer from the same problems as the liquid sample collection devices known in the art.

### Summary of the Invention

In accordance with a first aspect of the present invention, a liquid sample collection device comprises a housing including a liquid collection chamber, and a liquid supply conduit extending from an inlet port and in use generally downwardly to the collection chamber, wherein the collection chamber includes an air vent port located such that in use liquid supplied to the collection chamber through the liquid supply conduit displaces air through the air vent port, and wherein the liquid supply conduit follows a meandering path including at least one air trap section defined at the junction between portions of the conduit extending with upward and downward components respectively, whereby in use once the air vent port is closed, air is displaced along the liquid supply conduit until it is trapped by the air trap section thereby preventing further liquid flow through the conduit.

This invention provides a liquid sample collection device with no moving parts that contact the sample, capable of targeting and isolating a first portion of a liquid stream. The device comprises a meandering channel, which forms part of a liquid supply conduit supplying a liquid to be collected from an inlet port generally downwardly into a collection chamber. The collection chamber features an air vent port that connects the collection chamber to the atmosphere and acts as a pressure relief channel. As a liquid sample to be collected flows from the inlet port, through the meandering channel, and into the collection chamber, the air displaced escapes to the atmosphere via the vent port. However, once the vent port is obstructed, any additional liquid entering the collection chamber displaces air back up the meandering channel, where it becomes trapped in a local high point in the channel, thereby forming an air lock. The air lock prevents further liquid entering the collection chamber, and any additional liquid overflows the inlet port and is directed to waste.

In a particularly preferable embodiment, the air vent port is adapted to be closed, in use, by liquid collected in the collection chamber. In these embodiments, the air vent port may be positioned such that, as liquid collects within the collection chamber, the rising liquid level eventually obstructs the air vent port and engages the air lock. This embodiment is particularly preferable as it results in a device which can target and isolate a first portion of a liquid stream with no moving parts at all, reducing the complexity, manufacture cost and failure rate of the device. While this is preferable, other embodiments are foreseen in which the air vent port is obstructed by the manual closing of a valve along the air vent port. Advantageously, using a device in which the air vent port is obstructed by liquid collecting in the collection chamber results in a device which automatically engages an air lock, removing the need for manual human intervention. In embodiments where the air vent port is adapted to be closed, in use, by liquid collected in the collection chamber, the volume of the sample collected may be predetermined by the positioning of the vent port during manufacture of the device. When the liquid rises to the known level at which it obstructs the vent port, the air lock will take effect almost immediately resulting in a known volume of liquid being held within the collection chamber.

The liquid sample collection device comprises a second air trap section defined at a second junction between portions of the conduit extending with upward and downward components respectively. By providing a second air trap section, located at a second local high point, the device becomes resistant to shaking and overturning. In a case where the device is shaken or overturned once a sample has been collected and an air lock has taken effect, the airlock may move further up the meandering channel to the second air trap section, thereby maintaining an air lock within the device, and preventing escape of the collected sample and further liquid collection by the device. Advantageously, a device comprising two air trap sections would allow a user to turn the device upside down to remove all excess liquid from the device while maintaining the air lock.

Once a sample is collected, it is desirable to be able to conveniently access and remove the sample from the collection chamber. Therefore, preferably, the liquid sample collection device comprises a sample extraction port configured to allow access to the collection chamber, such that in use a liquid sample stored within the collection chamber may be extracted through the sample extraction port. While preferable, an extraction port is not necessary, and the sample may be extracted via the meandering channel by tipping of the device, or the sample may be tested in situ. An extraction port may simply allow the liquid to be poured out of the device, but preferably would allow the sample to be collected using a device, such as a syringe, without exposing it to contamination by the atmosphere. In order to allow sample extraction without contamination from the environment, it is preferable that the sample extraction port comprises an opening through the housing and extending into the collection chamber, wherein the opening is filled by a plug, such as a solid piece of rubber, adapted to allow a sample to be injected therethrough. A solid piece of rubber would form a membrane which could be pierced by a needle to allow extraction of the sample using a syringe. Once the needle is removed, the rubber would flex back, thereby blocking the channel formed by the needle. In order for the rubber to form a strong seal once a needle has been inserted and removed, it is preferable that the rubber has a Shore hardness equal to or less than A30. While an extraction port comprising a rubber membrane is preferable, other extraction ports are foreseen, such as a Luer lock or screw cap.

With a sample collected in the collection chamber, it may be desirable to mix the sample with an additive, either immediately after collection or at some point before extraction. Preferably, the liquid sample collection device comprises an additive port configured to allow an additive to be introduced into the collection chamber. While preferable, it is foreseen that devices may alternatively be used which contain an additive already within the collection chamber, ready for immediate mixing with a collected sample. Preferably an additive port comprises an opening through the housing and extending into the collection chamber, wherein the additive port interfaces with a blister pack located external to the housing. In such embodiments, the blister pack would be depressed by a user after collection of a sample to burst the blister and introduce its contents into the collection chamber via the opening in the housing. This is particularly preferable as it allows a user to conveniently mix their sample with an additive immediately after collection. While preferable, it is foreseen that other additive ports may be used. For example, a rubber membrane similar to the above described sample extraction port may be used. The rubber membrane could be pierced by a needle so that a syringe could be used to introduce an additive into the collection chamber. The rubber would flex back upon removal of the needle, thereby blocking the channel formed by the needle and ensuring that liquid within the collection chamber does not leak into the environment.

Preferably, the air vent port opens into the collection chamber through a side wall of the collection chamber. While preferable, it is also foreseen that the air vent port may open into the collection chamber through a top wall of the collection chamber.

Preferably, the device further comprises means for closing the vent port in an overturned configuration. Closing the vent port in an overturned configuration allows for an air lock which may be maintained when the device is overturned. The closing of the vent port may be achieved, for example, using a cap inserted into the vent port, or alternatively using a valve in the vent port which may be closed. However preferably, the vent port is closed in the overturned configuration by liquid in the collection chamber.

According to a second aspect of the present invention, a liquid sample collection system comprises at least two liquid sample collection devices according to the first aspect of the invention, wherein the at least two liquid sample collection devices are connected by an external liquid supply conduit configured to, in use, supply a liquid sample to be collected to the inlet port of the first of the at least two liquid sample collection devices, and when air trapped in the air trap section of the first liquid supply conduit prevents further liquid flowing through the liquid supply conduit of the first liquid sample collection device, supply further liquid sample to be collected to the inlet port of the second of the at least two liquid sample collection devices. Advantageously, using two liquid sample collection devices connected in series allows for a first portion and a second, subsequent portion of a liquid stream to be targeted and isolated.

Preferably, the external liquid supply conduit comprises a funnel, and preferably the funnel comprises a stem, an upstream part of which opens into the inlet port of the first liquid sample collection device, and a downstream part of which opens into the inlet port of the second liquid sample collection device at a position downstream of the inlet port of the first liquid sample collection device. By having the stem open into the inlet port of the first liquid sample collection device, and then downstream into the inlet port of the second liquid sample collection device, the second device may be filled with liquid which overflows the first liquid sample collection device, thereby targeting the second portion of the liquid stream without the use of any moving parts to divert the stream. While a funnel and stem are both preferable in forming the external liquid supply conduit, any apparatus capable of supplying a liquid flow to the first and then second device may be used, for example, a conduit comprising an internal valve located at a fork between conduit portions extending to the first and second devices.

### Brief Description of the Drawings

Some examples of liquid sample collection devices according to the invention will now be described with reference to the accompanying drawings in which:
Figure 1 is a perspective view of a liquid sample collection device according to a first comparative example;
Figure 2 is a cross-section through the liquid sample collection device of Figure 1;
Figure 3 is a cross-section through the liquid sample collection device of Figure 1, in use;
Figure 4 is cross-section through the liquid sample collection device of Figure 1 with corresponding funnel;
Figure 5 is a cross-section through two liquid sample collection devices of Figure 1, connected by an appropriate funnel;
Figure 6 is cross-section through a liquid sample collection device according to a second comparative example;
Figure 7 is cross-section through a liquid sample collection device according to a first embodiment;
Figure 8 is a perspective view of the liquid sample collection device of Figure 7, with its front face shown as transparent to reveal the internal features
Figures 9A to 9E are cross-sections through the liquid sample collection device of Figure 7, showing different stages during sample collection.

### Detailed Description

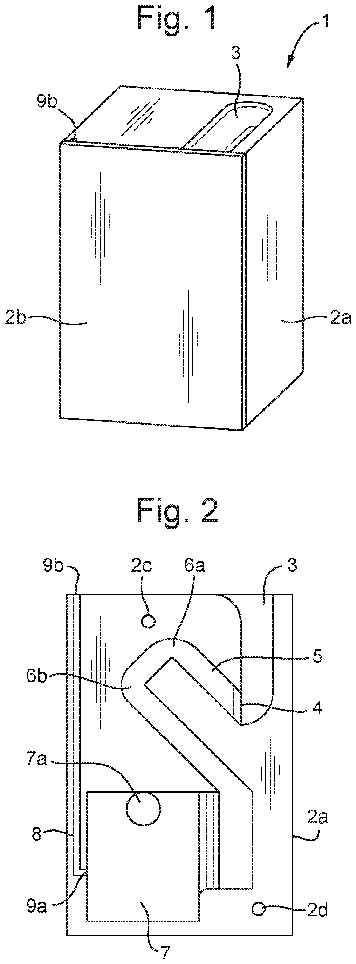

A liquid sample collection device 1 comprising a single meander is shown in Figures 1, and 2. The liquid sample collection device 1 comprises a solid cuboidal body 2a with dimensions of approximately 3 cm wide, 5 cm high and 4 cm deep. The device 1 also comprises a cover 2b which covers a front face of the body of the device 2a. The front face of the body of the device 2a has a series of recesses which partially define a number of features in its surface. These features include an inlet slot 3, a channel 5, a collection chamber 7, and pressure relief channel 8. The features partially defined by these recesses are completed and made internal to the device when the cover 2b is attached to the front of the body of the device 2a. The body of the device 2a also includes two location features 2c, 2d for aligning and attaching the cover 2b using corresponding location features (not shown) on the cover.

The body of the device 2a is a single piece of material, preferably formed into the desired shape in a single process. The body 2a is injection moulded using polypropylene. The cover 2b is a separate piece of polypropylene with an adhesive coating on one side. The device 1 is assembled by adhering the cover 2b to the front of the body of the device 2a. The location features 2c, 2d serve to align the cover 2b so that it adheres correctly.

The slot opening 3 in the upper surface of the body 2a of the device 2a forms the start of a liquid supply conduit for guiding liquid towards the collection chamber 7. The slot extends a substantial portion of the front-to-back depth of the body 2a, and extends down into the device by approximately 3 cm. In use, this slot funnels a liquid sample supplied into the slot towards an opening 4 of the channel 5 at a lower side of the slot.

The channel 5 is of substantially constant cross-section, the front-to-back depth, at 0.5 cm, being significantly less than the front-to-back depth of the slot 3, and the breadth of the channel being approximately 0.5 cm. The channel 5 proceeds from the opening 4 diagonally upwards and away from the slot 3 for approximately 1 cm at an angle of approximately 45° to the horizontal. The channel 5 then reaches a first turning point 6a where the channel changes direction by approximately 90° to proceed diagonally down and away from the slot 3. At the first turning point 6a the channel 5 is located entirely above the opening 4, at a high point relative to the rest of the channel 5; however, it is not located above the height of the top of the slot 3, i.e. the entrance to the liquid supply conduit. This first turning point 6a and the portions of channel extending either side of it form a first air trap section of the device.

As the channel proceeds diagonally down and away from the first turning point 6a, it reaches a second turning point 6b after approximately 0.5 cm. At the second turning point 6b the channel turns again by approximately 90° and proceeds diagonally down past a top corner of a collection chamber 7. The channel 5 then runs vertically, adjacent to the collection chamber 7. The channel 5 opens into the collection chamber through their adjacent running walls, thereby completing the liquid supply conduit.

The collection chamber 7 is a substantially cuboidal hollow chamber in the lower portion of the body 2a of the liquid sample collection device 1, having dimensions of approximately 1.5 cm wide, 20 cm high and 25 cm deep. In use, the collection chamber 7 stores a liquid sample collected by the device 1. The collection chamber is also connected to the pressure relief channel 8. The pressure relief channel 8 is narrower than the channel 5 and extends from an entrance 9a extending through a sidewall of the collection chamber, the entrance 9a being at a position between the top and bottom of the chamber, up through the body of the device 2a to a pressure relief channel exit 9b through the upper surface of the device, thereby connecting the collection chamber to the atmosphere.

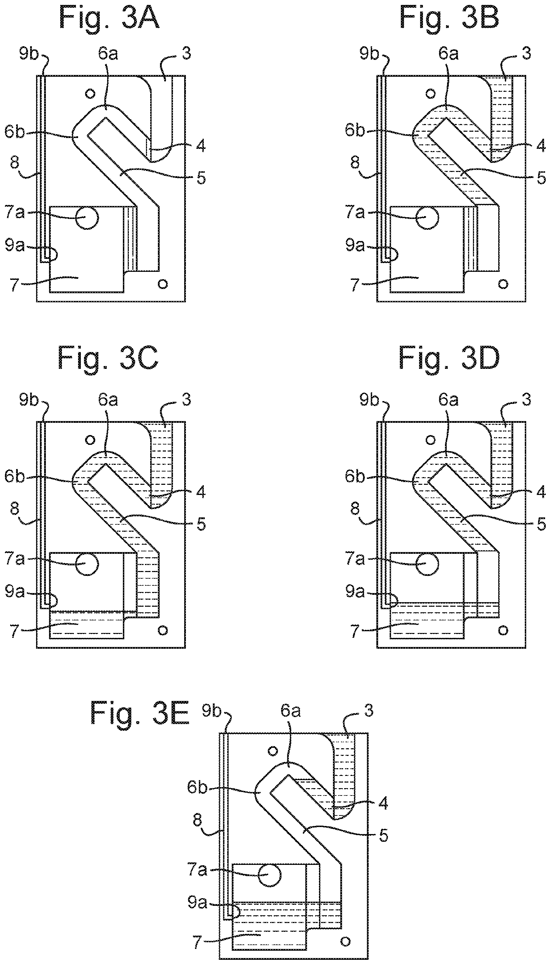

The process of collecting a sample using the device discussed above will now be described with reference to Figures 3A to 3E. The device is shown empty in Figure 3A, the liquid conduit, collection chamber 7, and pressure relief channel 8 being filled with air only. In use, a liquid sample to be collected enters the liquid sample collection device, via the slot 3. The liquid enters the channel 5 through the channel opening 4 at a lower side of the slot and, being urged along by further liquid supplied into the slot 3 behind, proceeds along the channel as shown in Figure 3B. The air present in the channel 5 prior to use is displaced along the channel by the advancing liquid. The pressure relief channel 8 allows the air being displaced by the increasing volume of liquid in the channel 5 to escape to the atmosphere, thereby maintaining atmospheric pressure within the device 1.

As shown in Figure 3C, the liquid sample proceeds along the channel 5 and into the collection chamber 7. As the liquid sample collects within the collection chamber 7, the liquid level rises continuing to displace air within the collection chamber 7 along the pressure relief channel 8. The liquid in the collection chamber 7 eventually rises until it is above the entrance 9a to the pressure relief channel 8, as shown in Figure 3D. At this point, the air within the collection chamber can no longer escape along the pressure relief channel 8. As liquid continues to flow into the collection chamber 7, air trapped within the device is now displaced back up the channel 5. The air reaches the first air trap section located at the first turning point 6a and becomes trapped in the high point formed by the channel 5, thereby forming an air lock, as shown in Figure 3E. With the trapped air forming an air lock, liquid entering through the slot 3 can no longer proceed through the channel 5, and instead overflows the slot and is directed to waste. In this way, a first portion of the liquid sample is collected and isolated from any remaining liquid.

As a result of this structure, the sample collected in the collection chamber 7 is of an approximately known volume. This is because the air lock always forms when the liquid level rises to a point just above the entrance 9a to the pressure relief channel 8. The whole device 1 may be scaled up or down in manufacture to collect a larger or smaller volume of liquid before the air lock takes effect. However, preferably, devices 1 are manufactured with the same dimensions for the liquid channel, collection chamber etc. and the volume of liquid collected is determined during manufacture by altering the height of the entrance 9a to the pressure relief channel 8 in the side wall of the collection chamber 7. That is to say, an increased volume of liquid may be collected by positioning the entrance 9a higher on the side wall of the collection chamber 7 and conversely, a smaller volume of liquid may be collected by positioning the entrance 9a lower on the side wall of the collection chamber 7.

Once the air lock has taken effect, the sample within the device 1 is substantially protected from contamination by the atmosphere. Provided that an amount of liquid remains within the slot, filling to at least above the entrance 4 to the channel 5, the sample is only exposed to the atmosphere via the pressure relief channel 8. In some embodiments of the invention (not shown), there may be provided means for sealing the pressure relief channel 8 once a sample has been collected. For example, a cap may be installed on the top of the device for blocking the relief channel.

The device 1 also comprises a sample extraction port 7a in the form of a frangible membrane which closes a circular opening through the rear of the device that opens into the collection chamber 7. The frangible membrane is a solid piece of rubber with a Shore hardness equal to or less than A30. The sample extraction port 7a may be pierced by a needle to allow extraction of a collected sample using, for example, a syringe, preferably without exposing the sample to the environment. Once the needle is removed from the extraction port 7a, the rubber flexes back to block the channel formed by the needle. In alternative examples (not shown), the sample extraction port 7a may be a screw cap opened to conveniently access and extract a sample stored in the collection chamber 7, or may be a Luer lock for interfacing with a syringe.

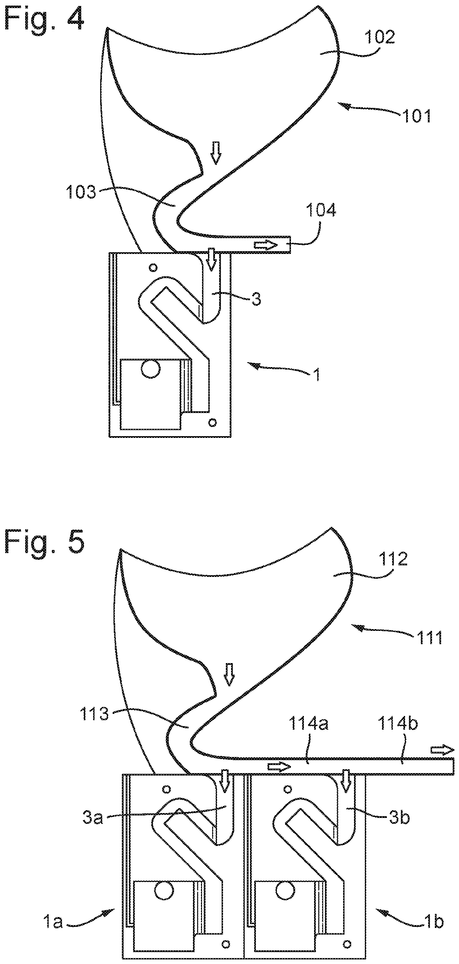

A funnel 101 suitable for use with the liquid sample collection device 1 is shown in Figure 4. In use, the funnel is mounted on top of the device and guides liquid to be collected towards the slot 3 in the upper surface of the device 1. The funnel has a mouth 102 that collects the liquid sample and guides it towards the stem 103. The stem 103 of the funnel 101 guides liquid towards the slot 3 from one side of the slot opening. Liquid flowing through the funnel enters the slot and fills the device, as described above, until the air lock prevents further filling. The slot 3 then overflows and any further liquid entering the funnel 101 is directed to waste via a runoff portion 104 of the funnel. The runoff portion of the funnel is located at the opposite side of the slot 3 to the stem 103.

An alternative funnel 111 is shown in Figure 5. This funnel is shaped to connect with two devices 1a, 1b to allow the devices to target two separate portions of the liquid sample. The funnel 111 guides liquid toward the first device in an identical manner to the funnel 101 shown in Figure 4. The first device 1a collects the first portion of the liquid sample before an air lock prevents further filling. The air lock causes further liquid entering the funnel 111 to overflow the slot 3a and enter the run off 114a of the funnel 111. This runoff 114a then directs the overflow liquid to a second device 1b located next to the first device 1a. The second device 1b works in a manner identical to the first device 1a, but in this configuration collects a second portion of the liquid sample and isolates this from any further liquid entering the funnel 111. When the air lock takes effect in the second device 1b and the slot 3b overflows, any further liquid enters a second runoff portion 114b and is directed to waste.

It will be appreciated that any number of devices may be connected in the way described above to target different portions of the liquid sample. Further, the devices may collect the same or different volumes of liquid depending on the positioning of the entrance 9a of the pressure relief channel 8 within each device.

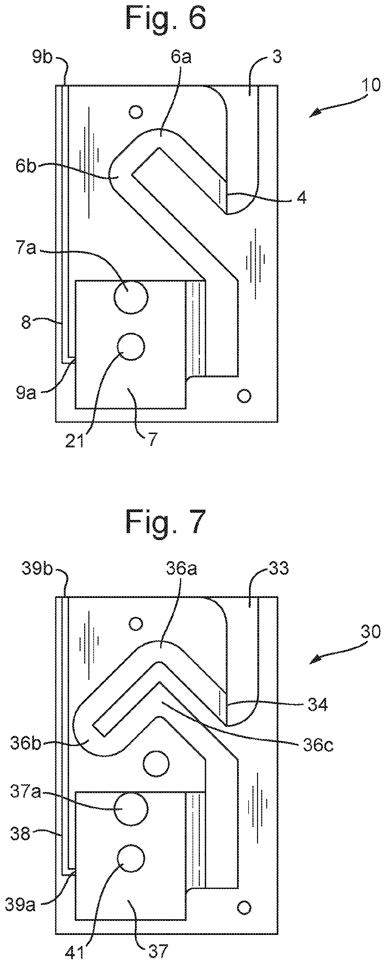

In a second comparative example, shown in Figure 6, the liquid sample collection device 10 comprises an additive port 21. In this comparative example, those parts that are the same as in the first comparative examplewill not be described again. The additive port 21 is an opening through the rear of the device that opens into the collection chamber 37. The additive port interfaces with a blister pack (not shown) on the rear of the device. The user may depress the blister pack from the rear of the device, bursting it and releasing an additive into the collection chamber 37. The use of a blister pack would allow a user to mix a sample with an additive immediately after collection. In an alternative example (not shown), the additive port 21 comprises an opening through the rear of the device that is filled with a solid piece of rubber with a Shore hardness of less than A30. In such comparative examples, a needle and syringe may be used to inject an additive into the collection chamber 27. The rubber forming the additive port 21 would flex back upon removal of the needle to block the channel formed by the needle and prevent any liquid leaking through the additive port. In a further alternative (not shown), the additive port 21 is a Luer lock suitable for interfacing with a syringe which may be used to introduce an additive into the collection chamber 27.

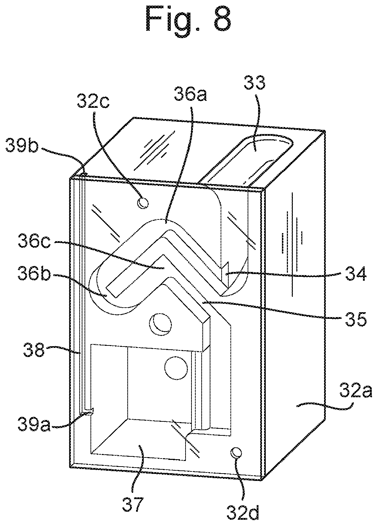

A liquid collection device 30 comprising a double meander according to a first embodiment is shown in Figures 7 and 8. The liquid collection device comprises a slot opening 33 in the upper surface of the device with an opening 34 in its lower side, as in the first comparative example. This forms the start of the liquid supply conduit. The opening 34 connects to a channel 35 which proceeds diagonally upwards and away from the slot 33 at an angle of approximately 45° to the horizontal. The channel 35 reaches a first turning point 36a where it turns through approximately 90° to proceed diagonally downwards and away from the slot 33. The turning point 36a forming a first high point in the channel 35, in which the channel 35 is entirely above the opening 34 to the channel 35. The first turning point 36a and the portions of channel extending either side of it form a first air trap section in the device. The channel 35 then reaches a second turning point 36b at which it turns and proceeds back on itself; running parallel and beneath the portion of the channel 35 between the first and second turning points 36a, 36b. The channel 35 then reaches a third turning point 36c, at which the channel 35 is entirely above the second turning point 36b, where it turns through approximately 90° and proceeds diagonally downward; running parallel and beneath the portion of the channel 35 between the first turning point 36a and the opening 34. The third turning point 36c forms a local high point relative to the portions of the channel 35 on either side. The third turning point 36c and the portions of channel extending either side of it form a second air trap section in the device. The channel 35 then runs between the third turning point 36c and a top corner of a collection chamber 37 in the lower portion of the device 30.

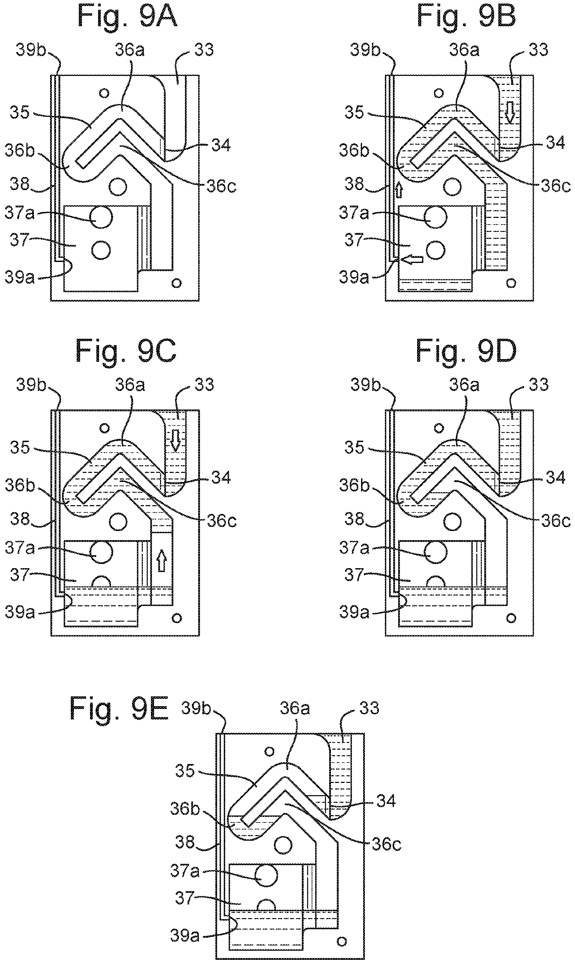

The process of collecting a sample using the double meander device discussed above will now be described with reference to Figures 9A to 9d. The device is shown empty in Figure 9A, the liquid conduit, collection chamber 37, and pressure relief channel 38 being filled with air only. In use, a liquid enters the slot 33 and passes into the channel 35 via the opening 34. The liquid is urged along the channel 35 by the liquid collecting in the slot 33 behind, displacing the air occupying the channel along and out of the system via the pressure relief channel 38. The liquid proceeds along the channel 35 and into the collection chamber 37 which it begins to fill, as shown in Figure 9B. Once the collection chamber 37 fills with liquid to a point where the liquid level is above the entrance 39a to the pressure relief channel 38, as shown in Figure 9C, the gas within the collection chamber can no longer escape to the atmosphere and is displaced back up the channel 35. The gas becomes trapped in the local high point formed by the third turning point 36c, thereby forming an air lock in the channel 35, as shown in Figure 9D, preventing any additional liquid entering the collection chamber 37.

Advantageously, because the double meander embodiment features two high points formed by turning points 36a and 36c, the device becomes resistant to shaking and overturning after collection of a sample. If the device is shaken or rotated through 360° the air lock may move from the third turning point 36c to the first turning point 36a, as shown in Figure 9E, thereby maintaining the air lock. The device may also be overturned without breaking the air lock provided that the pressure relief channel remains blocked in the overturned position. If the pressure relief channel remains blocked, the air trapped in the third turning point may move to the second turning point, which in an overturned orientation forms a local high point. Preferably, the pressure relief channel is positioned such that in the overturned position the liquid level remains above the entrance to the pressure relief channel, i.e. the pressure relief channel is located above the median line of the collection chamber. Alternatively, the pressure relief channel may be blocked manually, for example using a cap, so that in an overturned position air cannot enter the collection chamber through the pressure relief channel.

## Claims

1. A liquid sample collection device comprising:
a housing (2a, 2b) including a liquid collection chamber (37), and a liquid supply conduit (35) extending from an inlet port (33) and in use generally downwardly to the collection chamber (37), wherein the collection chamber (37) includes an air vent port (39a) located such that in use liquid supplied to the collection chamber (37) through the liquid supply conduit (35) displaces air through the air vent port (39a), and wherein the liquid supply conduit (35) follows a meandering path including a first air trap section (36c) defined at the junction between portions of the conduit extending with upward and downward components respectively, whereby in use once the air vent port (39a) is closed, air is displaced along the liquid supply conduit (35) until it is trapped by the first air trap section (39a) thereby preventing further liquid flow through the conduit (35), and **characterised in that** the device comprises a second air trap (36a) section defined at a second junction between portions of the conduit extending with upward and downward components respectively.

2. The liquid sample collection device according to claim 1, wherein the air vent port (39a) is adapted to be closed, in use, by liquid collected in the collection chamber.

3. The liquid sample collection device according to any of the preceding claims, further comprising a sample extraction port (37a) configured to allow access to the collection chamber (37), such that in use a liquid sample stored within the collection chamber (37) may be extracted through the sample extraction port (37a).

4. The liquid sample collection device according to claim 3, wherein the sample extraction port (37a) comprises an opening through the housing and extending into the collection chamber (37), and wherein the opening is filled by a plug, such as a solid piece of rubber adapted to allow a sample to be injected therethrough.

5. The liquid sample collection device according to claim 4, wherein the rubber has a Shore hardness equal to or less than A30.

6. The liquid sample collection device according to any of the preceding claims, further comprising an additive port (41) configured to allow an additive to be introduced into the collection chamber (37).

7. The liquid sample collection device according to claim 6, wherein the additive port (41) comprises an opening through the housing (2a, 2b) and extending into the collection chamber (37), and wherein the additive port (41) interfaces with a blister pack located external to the housing.

8. The liquid sample collection device according to at least claim 2, wherein the air vent port (39a) opens into the collection chamber (37) through a side wall of the collection chamber (37).

9. The liquid sample collection device according to any of the preceding claims, further comprising means for closing the vent port when in an overturned orientation.

10. A liquid sample collection system comprising at least two liquid sample collection devices according to any of the preceding claims, wherein the at least two liquid sample collection devices are connected by an external liquid supply conduit (103) configured to, in use, supply a liquid sample to be collected to the inlet port (33) of the first of the at least two liquid sample collection devices, and when air trapped in the first air trap section of the first liquid supply conduit prevents further liquid flowing through the liquid supply conduit (35) of the first liquid sample collection device, supply further liquid sample to be collected to the inlet port (33) of the second of the at least two liquid sample collection devices.

11. The liquid sample collection system according to claim 10, wherein the external liquid supply conduit comprises a funnel (101).

12. The liquid sample collection system according to claim 11, wherein the funnel (101) comprises a stem, wherein the stem feeds into the inlet port (33) of the first liquid sample collection device, and into the inlet port (33) of the second liquid sample collection device at a position downstream of the inlet port (33) of the first liquid sample collection device.

## Patentansprüche

1. Sammelvorrichtung für eine Flüssigkeitsprobe, Folgendes umfassend:
ein Gehäuse (2a, 2b), einschließlich einer Flüssigkeitssammelkammer (37), und eine Flüssigkeitszufuhrleitung (35), die sich von einer Einlassöffnung (33) und in Verwendung im Allgemeinen abwärts zu der Sammelkammer (37) hin erstreckt, wobei die Sammelkammer (37) eine Entlüftungsöffnung (39a) beinhaltet, die derart angeordnet ist, dass in Verwendung Flüssigkeit, die an die Sammelkammer (37) durch die Flüssigkeitszufuhrleitung (35) zugeführt wird, Luft durch die Entlüftungsöffnung (39a) verdrängt, und wobei die Flüssigkeitszufuhrleitung (35) einem schlängelnden Pfad, einschließlich eines ersten Geruchsverschlussbereichs (36c), folgt, der an der Verbindung zwischen Abschnitten der Leitung definiert ist, die sich mit aufwärtsgerichteten beziehungsweise abwärtsgerichteten Komponenten erstrecken, wodurch Luft, sobald die Entlüftungsöffnung (39a) geschlossen ist, in Verwendung entlang der Flüssigkeitszufuhrleitung (35) verdrängt wird, bis sie durch den ersten Geruchsverschlussbereich (39a) gefangen wird, wobei dadurch ein weiterer Flüssigkeitsstrom durch die Leitung (35) verhindert wird, und **dadurch gekennzeichnet, dass** die Vorrichtung einen zweiten Geruchsverschluss(36a)bereich umfasst, der an einer zweiten Verbindung zwischen Abschnitten der Leitung, die sich mit aufwärtsgerichteten beziehungsweise abwärtsgerichteten Komponenten erstrecken, definiert ist.

2. Sammelvorrichtung für eine Flüssigkeitsprobe nach Anspruch 1, wobei die Entlüftungsöffnung (39a) angepasst ist, um in Verwendung durch die in der Sammelkammer gesammelte Flüssigkeit geschlossen zu werden.

3. Sammelvorrichtung für eine Flüssigkeitsprobe nach einem der vorhergehenden Ansprüche, ferner umfassend eine Probenentnahmeöffnung (37a), die konfiguriert ist, um einen Zugang zu der Sammelkammer (37) derart zu ermöglichen, dass eine Flüssigkeitsprobe, die innerhalb der Sammelkammer (37) gespeichert ist, in Verwendung durch die Probenentnahmeöffnung (37a) entnommen werden kann.

4. Sammelvorrichtung für eine Flüssigkeitsprobe nach Anspruch 3, wobei die Probenentnahmeöffnung (37a) einen Eingang durch das Gehäuse und sich in die Sammelkammer (37) erstreckend umfasst, und wobei der Eingang durch einen Stopfen gefüllt ist, wie etwa ein festes Stück Kautschuk, das befestigt ist, um zu ermöglichen, dass eine Probe dahindurch eingespeist werden kann.

5. Sammelvorrichtung für eine Flüssigkeitsprobe nach Anspruch 4, wobei der Kautschuk eine Shore-Härte aufweist, die höchstens A30 entspricht.

6. Sammelvorrichtung für eine Flüssigkeitsprobe nach einem der vorhergehenden Ansprüche, ferner umfassend eine Zusatzstofföffnung (41), die konfiguriert ist, um zu ermöglichen, dass ein Zusatzstoff in die Sammelkammer (37) eingeführt wird.

7. Sammelvorrichtung für eine Flüssigkeitsprobe nach Anspruch 6, wobei die Zusatzstofföffnung (41) einen Eingang durch das Gehäuse (2a, 2b) und sich in die Sammelkammer (37) erstreckend umfasst, und wobei die Zusatzstofföffnung (41) an eine Blisterverpackung, die außerhalb des Gehäuses angeordnet ist, ankoppelt.

8. Sammelvorrichtung für eine Flüssigkeitsprobe nach wenigstens Anspruch 2, wobei sich die Entlüftungsöffnung (39a) durch eine Seitenwand der Sammelkammer (37) in die Sammelkammer (37) hinein öffnet.

9. Sammelvorrichtung für eine Flüssigkeitsprobe nach einem der vorhergehenden Ansprüche, ferner umfassend ein Mittel zum Schließen der Entlüftungsöffnung, wenn sie sich in einer umgekippten Ausrichtung befindet.

10. Sammelsystem für eine Flüssigkeitsprobe, umfassend wenigstens zwei Sammelvorrichtungen für eine Flüssigkeitsprobe nach einem der vorhergehenden Ansprüche, wobei die wenigstens zwei Sammelvorrichtungen für eine Flüssigkeitsprobe durch eine äußere Flüssigkeitszufuhrleitung (103) verbunden sind, die konfiguriert ist, um, in Verwendung eine zu sammelnde Flüssigkeitsprobe zu der Einlassöffnung (33) der ersten der wenigstens zwei Sammelvorrichtungen für eine Flüssigkeitsprobe zuzuführen, und, wenn Luft, die in dem ersten Geruchsverschlussbereich der ersten Flüssigkeitszufuhrleitung gefangen ist, verhindert, dass weitere Flüssigkeit durch die Flüssigkeitszufuhrleitung (35) der ersten Sammelvorrichtung für eine Flüssigkeitsprobe fließt, eine weitere zu sammelnde Flüssigkeitsprobe an die Einlassöffnung (33) der zweiten der wenigstens zwei Sammelvorrichtungen für eine Flüssigkeitsprobe zuzuführen.

11. Sammelsystem für eine Flüssigkeitsprobe nach Anspruch 10, wobei die äußere Flüssigkeitszufuhrleitung einen Trichter (101) umfasst.

12. Sammelsystem für eine Flüssigkeitsprobe nach Anspruch 11, wobei der Trichter (101) einen Schaft umfasst, wobei der Schaft in die Einlassöffnung (33) der ersten Sammelvorrichtung für eine Flüssigkeitsprobe und in die Einlassöffnung (33) der zweiten Sammelvorrichtung für eine Flüssigkeitsprobe an einer der Einlassöffnung (33) der ersten Sammelvorrichtung für eine Flüssigkeitsprobe nachgelagerten Position einleitet.

## Revendications

1. Dispositif de collecte d'échantillon liquide comprenant :
un boîtier (2a, 2b) comportant une chambre de collecte de liquide (37) et un conduit d'alimentation en liquide (35) s'étendant depuis un orifice d'entrée (33) et utilisé de manière générale vers le bas vers la chambre de collecte (37), la chambre de collecte (37) comportant un orifice de ventilation (39a) situé de telle sorte que, lors de l'utilisation, un liquide alimenté à la chambre de collecte (37) à travers le conduit d'alimentation en liquide (35) déplace de l'air par l'orifice de ventilation (39a), et dans lequel le conduit d'alimentation en liquide (35) suit un chemin sinueux comportant une première section de piège à air (36c) définie à la jonction entre des parties du conduit s'étendant respectivement avec des composants ascendants et descendants, de sorte que lors de l'utilisation, une fois que l'orifice de ventilation (39a) est fermé, de l'air est déplacé le long du conduit d'alimentation en liquide (35) jusqu'à ce qu'il soit piégé par la première section de piège à air (39a) empêchant ainsi un écoulement supplémentaire de liquide à travers le conduit (35), et **caractérisé en ce que** le dispositif comprend une seconde section de piège à air (36a) définie au niveau d'une seconde jonction entre des parties du conduit s'étendant respectivement avec des composants ascendants et descendants.

2. Dispositif de collecte d'échantillon liquide selon la revendication 1, dans lequel l'orifice de ventilation (39a) est adapté pour être fermé, lors de l'utilisation, par le liquide collecté dans la chambre de collecte.

3. Dispositif de collecte d'échantillon liquide selon l'une quelconque des revendications précédentes, comprenant en outre un orifice d'extraction d'échantillon (37a) conçu pour permettre l'accès à la chambre de collecte (37), de telle sorte que lors de l'utilisation, un échantillon liquide stocké à l'intérieur de la chambre de collecte (37) peut être extrait à travers l'orifice d'extraction d'échantillon (37a).

4. Dispositif de collecte d'échantillon liquide selon la revendication 3, dans lequel l'orifice d'extraction d'échantillon (37a) comprend une ouverture à travers le boîtier et qui s'étend dans la chambre de collecte (37), et dans lequel l'ouverture est remplie par un bouchon, tel qu'une pièce en caoutchouc solide adapté pour permettre l'injection d'un échantillon à travers celle-ci.

5. Dispositif de collecte d'échantillon liquide selon la revendication 4, dans lequel le caoutchouc a une dureté Shore égale ou inférieure à A30.

6. Dispositif de collecte d'échantillon liquide selon l'une quelconque des revendications précédentes, comprenant en outre un orifice d'additif (41) conçu pour permettre à un additif d'être introduit dans la chambre de collecte (37).

7. Dispositif de collecte d'échantillon liquide selon la revendication 6, dans lequel l'orifice d'additif (41) comprend une ouverture à travers le boîtier (2a, 2b) et s'étend dans la chambre de collecte (37), et dans lequel l'orifice d'additif (41) est en interface avec un emballage-coque situé à l'extérieur du boîtier.

8. Dispositif de collecte d'échantillon liquide selon au moins la revendication 2, dans lequel l'orifice de ventilation (39a) débouche dans la chambre de collecte (37) à travers une paroi latérale de la chambre de collecte (37).

9. Dispositif de collecte d'échantillon liquide selon l'une quelconque des revendications précédentes, comprenant en outre un moyen pour fermer l'orifice de ventilation lorsqu'il est dans une orientation retournée.

10. Système de collecte d'échantillon liquide comprenant au moins deux dispositifs de collecte d'échantillon liquide selon l'une quelconque des revendications précédentes, dans lequel les au moins deux dispositifs de collecte d'échantillon liquide sont raccordés par un conduit d'alimentation en liquide externe (103) conçu pour, lors de l'utilisation, alimenter un échantillon de liquide à collecter à l'orifice d'entrée (33) du premier des au moins deux dispositifs de collecte d'échantillon de liquide, et lorsque de l'air piégé dans la première section de piège à air du premier conduit d'alimentation en liquide empêche tout autre écoulement de liquide à travers le conduit d'alimentation en liquide (35) du premier dispositif de collecte d'échantillon liquide, alimenter un autre échantillon liquide à collecter à l'orifice d'entrée (33) du second des au moins deux dispositifs de collecte d'échantillon liquide.

11. Système de collecte d'échantillon liquide selon la revendication 10, dans lequel le conduit d'alimentation en liquide externe comprend un entonnoir (101).

12. Système de collecte d'échantillon liquide selon la revendication 11, dans lequel l'entonnoir (101) comprend une tige, la tige se prolongeant dans l'orifice d'entrée (33) du premier dispositif de collecte d'échantillon liquide et dans l'orifice d'entrée (33) du second dispositif de collecte d'échantillon liquide en une position en aval de l'orifice d'entrée (33) du premier dispositif de collecte d'échantillon liquide.
